# EUROPEAN PATENT SPECIFICATION

(11) **EP 3 307 049 B1**
(45) Date of publication and mention of the grant of the patent: **14.02.2024**
(21) Application number: 16812374.3
(22) Date of filing: 15.06.2016
(51) Int. Cl.: A01B 79/02, A01C 5/08, A01C 15/00, A01C 21/00, A01C 23/02, G01N 33/24, G01N 21/55, G01N 21/31

(54) **SYSTEMS, METHODS, AND APPARATUS FOR AGRICULTURAL LIQUID APPLICATION**
SYSTEME, VERFAHREN UND VORRICHTUNG ZUM AUFBRINGEN VON LANDWIRTSCHAFTLICHER FLÜSSIGKEIT
SYSTÈMES, PROCÉDÉS ET APPAREIL POUR APPLICATION DE LIQUIDE AGRICOLE

(30) Priority: 15.06.2015 US 201562175920 P; 18.09.2015 US 201562220576 P; 18.01.2016 US 201662280085 P; 18.01.2016 US 201662279995 P
(43) Date of publication of application: 18.04.2018
(73) Proprietor: Precision Planting LLC, Tremont, IL 61568 (US)
(72) Inventor: LEVY, Kent, Morton, IL 61550 (US); RADTKE, Ian, Washington, IL 61571 (US); LEMAN, Tracy, Eureka, IL 61530 (US)
(74) Representative: AGCO Intellectual Property Department
(86) International application number: PCT/US2016/037701
(87) International publication number: WO 2016/205421

(56) References cited:
- US-A- 4 773 340
- US-A- 5 351 635
- US-A1- 2005 045 080
- US-A1- 2008 006 189
- US-A1- 2009 120 340
- US-A1- 2012 137 942
- US-A1- 2012 167 809
- US-A1- 2012 234 218
- US-A1- 2012 234 218
- US-A1- 2015 144 042
- US-B1- 6 382 114
- US-B2- 6 918 342

## Description

### BACKGROUND

In recent years, the availability of advanced location-specific agricultural application and measurement systems (used in so-called "precision farming" practices) has increased grower interest in determining spatial variations in soil properties and in varying input application variables (e.g. planting depth) and fertilizer and other liquid applications in light of such variations and at the appropriate location during the planting operation. However, the available mechanisms for measuring soil properties are not effectively locally made throughout the field or not made at the same time as an input operation (e.g. planting). Additionally, commercial solutions for applying liquid have included applying liquid on top of seeds in the planting trench, which may cause deleterious effects such as "burning" (i.e., over-fertilizing) seed. Other liquid application solutions have included opening a separate trench in the soil surface (disposed between the planting trenches opened by the row unit) and depositing liquid in the separate vertical trench, which may result in underutilization of applied fertilizer.

Thus there is a need in the art for effectively applying liquid during the planting operation.

U.S. Patent Application Publication No. US 2012/023 4218 A1 discloses apparatus on a planter row unit for delivering liquid fertilizer to the bottom of a trench. The apparatus includes a single furrow opening disc and a fertilizer feed tube shoe biased by a coil spring both against the disc and the bottom of a furrow created by the disc. A fertilizer feed tube is mounted to a reward surface of the fertilizer feed tube shoe.

### SUMMARY

In accordance with the invention, there is provided a liquid application apparatus for applying liquid to soil adjacent to a trench as set out in claim 1. Further optional features of the liquid application according to the invention are set out in the claims dependent on claim 1.

### DESCRIPTION OF THE DRAWINGS

FIG. 1 is a top view of an example of an agricultural planter.
FIG. 2 is a side elevation view of an example of a planter row unit.
FIG. 3A is a left side perspective view of an embodiment of an adjacent trench liquid delivery system.
FIG. 3B is a front perspective view of the adjacent trench liquid delivery system of FIG. 3A
FIG. 3C is a side elevation view of the adjacent trench liquid delivery system of FIG 3A.
FIG. 3D is a side elevation view of the adjacent trench liquid delivery system of FIG 3A disposed at a rearward angle away from a direction of travel
FIG. 3E is a side elevation view of another embodiment of the adjacent trench liquid delivery system having a doglegged downwardly extending element.
FIG. 4A is a side elevation view of an embodiment of an assembly showing the adjacent trench liquid delivery system of FIG. 3A mounted forward of a closing assembly.
FIG. 4B is a top plan view of the assembly of FIG. 4A.
FIG. 5A is a perspective view of another embodiment of an assembly showing the adj acent trench liquid delivery system of FIG. 3E mounted on a mounting bracket rearward of a closing assembly.
FIG. 5B is top plan view of the adjacent trench liquid delivery system of FIG. 5A.
FIG. 5C is a perspective view of another embodiment of a mounting bracket for mounting the adjacent trench liquid delivery system to the assembly of FIG. 5A.
FIG. 5D is a perspective view of the assembly and mounting bracket of FIG. 5C as part of a leveling system.

### DESCRIPTION

### Depth Control and Soil Monitoring Systems

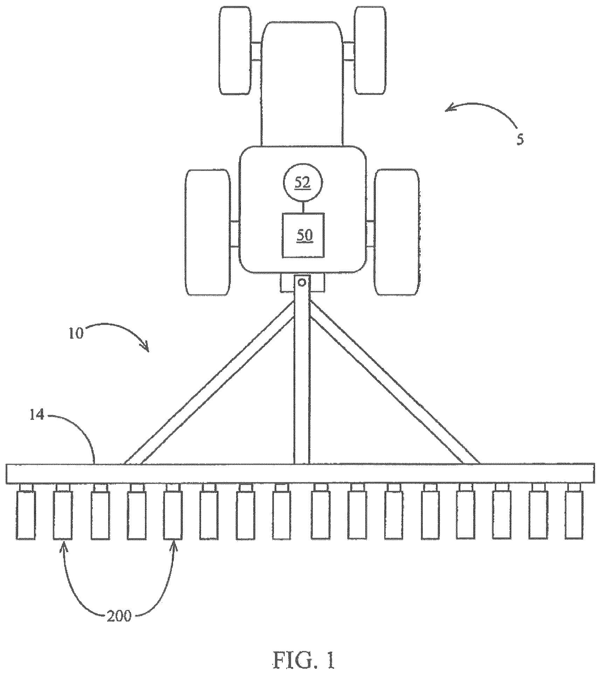

Referring now to the drawings, wherein like reference numerals designate identical or corresponding parts throughout the several views, FIG. 1 illustrates a tractor 5 drawing an agricultural implement, e.g., a planter 10, comprising a toolbar 14 operatively supporting multiple row units 200. An implement monitor 50 preferably including a central processing unit ("CPU"), memory and graphical user interface ("GUI") (e.g., a touch-screen interface) is preferably located in the cab of the tractor 5. A global positioning system ("GPS") receiver 52 is preferably mounted to the tractor 5.

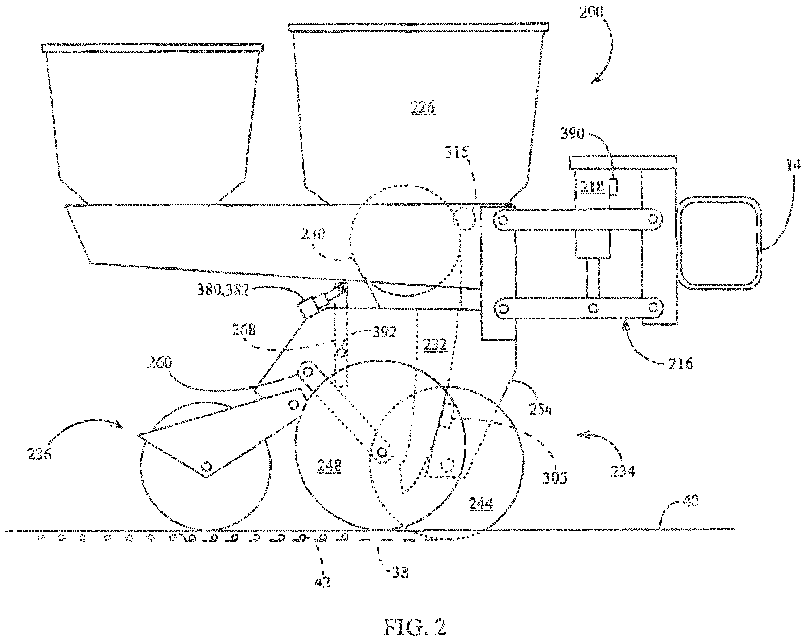

Turing to FIG. 2, a row planter unit 200 is illustrated in which the row unit 200 is a planter row unit. The row unit 200 is preferably pivotally connected to the toolbar 14 by a parallel linkage 216. An actuator 218 is preferably disposed to apply lift and/or downforce on the row unit 200. A solenoid valve 390 is preferably in fluid communication with the actuator 218 for modifying the lift and/or downforce applied by the actuator. An opening system 234 preferably includes two opening discs 244 rollingly mounted to a downwardly-extending shank 254 and disposed to open a v-shaped trench 38 in the soil 40. A pair of gauge wheels 248 is pivotally supported by a pair of corresponding gauge wheel arms 260. The height of the gauge wheels 248 relative to the opener discs 244 sets the depth of the trench 38. A depth adjustment rocker 268 limits the upward travel of the gauge wheel arms 260 and thus the upward travel of the gauge wheels 248. A depth adjustment actuator 380 is preferably configured to modify a position of the depth adjustment rocker 268 and thus the height of the gauge wheels 248. The actuator 380 is preferably a linear actuator mounted to the row unit 200 and pivotally coupled to an upper end of the rocker 268. In some embodiments the depth adjustment actuator 380 comprises a device such as that disclosed in International Patent Application No. PCT/US2012/035585 ("the '585 application"). An encoder 382 is preferably configured to generate a signal related to the linear extension of the actuator 380. It should be appreciated that the linear extension of the actuator 380 is related to the depth of the trench 38 when the gauge wheel arms 260 are in contact with the rocker 268. A downforce sensor 392 is preferably configured to generate a signal related to the amount of force imposed by the gauge wheels 248 on the soil 40; in some examples the downforce sensor 392 comprises an instrumented pin about which the rocker 268 is pivotally coupled to the row unit 200, such as those instrumented pins disclosed in Applicant's U.S. Patent Application No. 12/522,253.

Continuing to refer to FIG. 2, a seed meter 230 such as that disclosed in Applicant's International Patent Application No. PCT/US2012/030,192 is preferably disposed to deposit seeds 42 from a hopper 226 into the trench 38, e.g., through a seed tube 232 disposed to guide the seeds toward the trench. In some examples, instead of a seed tube 232, a seed conveyor is implemented to convey seeds from the seed meter to the trench at a controlled rate of speed as disclosed in U.S. Patent Application Serial No. 14/347,902 and/or U.S. Patent No. 8,789,482. In such examples, a bracket is preferably configured to mount the seed firmer to the shank 254 via sidewalls extending laterally around the seed conveyor, such that the seed firmer is disposed behind the seed conveyor to firm seeds into the soil after they are deposited by the seed conveyor. In some examples, the meter is powered by an electric drive 315 configured to drive a seed disc within the seed meter. In other embodiments, the drive 315 may comprise a hydraulic drive configured to drive the seed disc. A seed sensor 305 (e.g., an optical or electromagnetic seed sensor configured to generate a signal indicating passage of a seed) is preferably mounted to the seed tube 232 and disposed to send light or electromagnetic waves across the path of seeds 42. A closing system 236 including one or more closing wheels 238 is pivotally coupled to the row unit 200 and configured to close the trench 38.

### Adjacent Trench Liquid Delivery

There can be a need for delivering liquid adjacent the trench. For example, liquid in the sidewall of the trench can be used by a plant after germination. As the plant grows, this liquid will be consumed by the early growing plant. As the root structure develops and spreads out, the next stage of the plant can access the liquid applied adjacent to the trench. A benefit of having the additional source of fertilizer rather than all of the fertilizer for all growing stages is that the amount of fertilizer for all stages could be too much for the seed resulting in "burning".

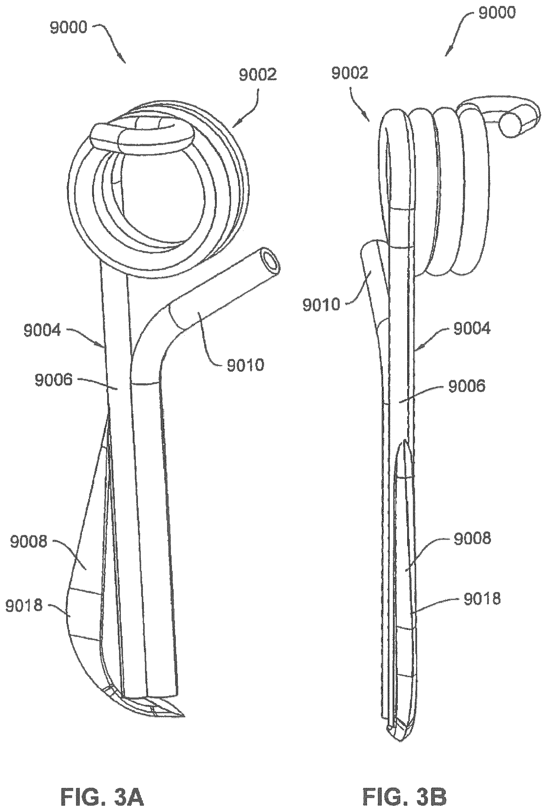

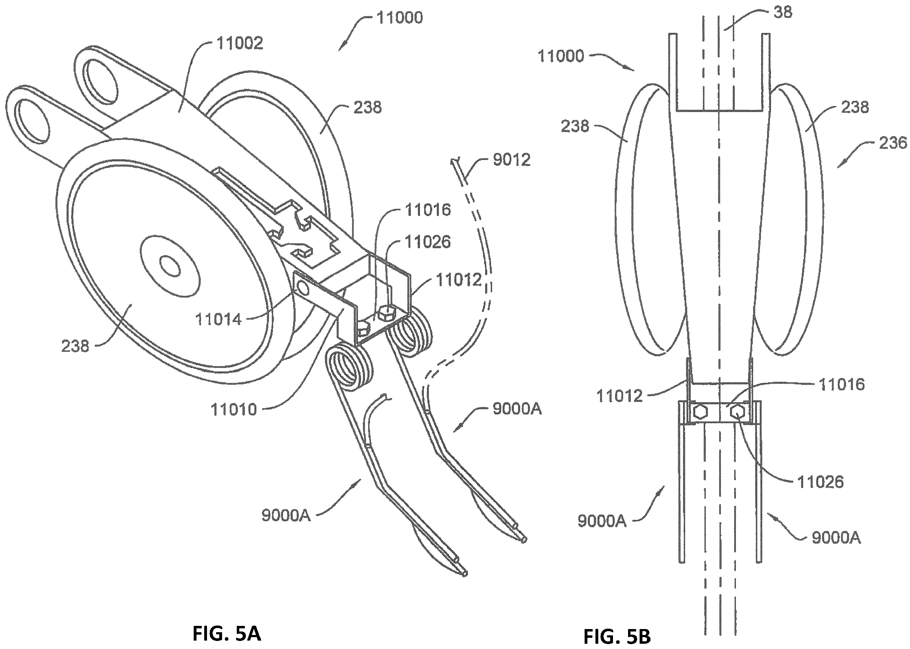

One embodiment of an adjacent trench liquid delivery system 9000 is shown in FIGs. 3A-C. The delivery system 9000 includes a relief element 9002 and a downwardly extending element 9004. The downwardly extending element 9004 includes a rigid leg 9006 to which is secured a knife 9008 positioned on a forward portion (i.e., toward the direction of travel of the row unit as indicated by arrow 9003 in FIGs.5A-5B). A liquid delivery tube 9010 is positioned on a rearward portion of the rigid leg 9006 (i.e., away from the direction of travel of the row unit). Fluid is communicated from a liquid source (not shown) to the liquid delivery tube 9010 via a liquid distribution tube 9012 (3C-3E) which couples to the liquid delivery tube 9010 via a coupler 9014 or other suitable connecting means. The liquid can be communicated via gravity feed or under pressure, such as with a pump.

The relief element 9002 allows the downwardly extending element 9004 to flex rearwardly and side-to-side if the downwardly extending element encounters an obstruction such as a rock as the row unit travels forwardly through the field and it also permits the downwardly extending element 9004 to shed foreign debris that may build up on the cutting edge 9018 of the knife 9008. In one embodiment, relief element 9002 comprises a coil spring with the rigid leg 9006 comprising one leg of the coil spring rod and the other end of the coil spring rod is bent outwardly forming a horizontal loop 9016 for receiving a mounting bolt for securing to a mounting plate as shown in FIG. 5A (discussed later).

The knife 9008 may be convex to the direction of travel. This convex shape helps avoid pinching debris between the cutting edge 9018 of the knife 9008 and the soil surface.

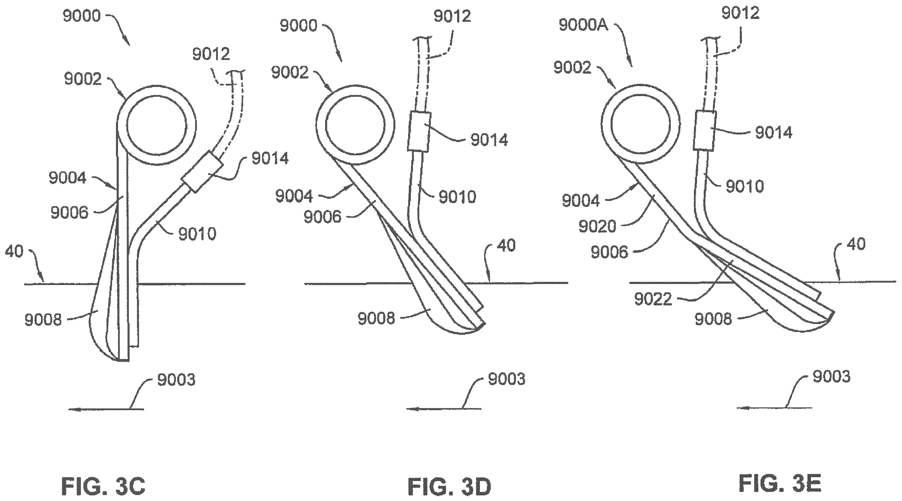

The adjacent trench liquid delivery system 9000 may be mounted (discussed later) such that the downwardly extending element 9004 is disposed substantially vertically with respect to the soil surface 40 as shown in FIG. 3C. The adjacent trench liquid delivery system 9000 may be disposed such that it angles rearwardly with respect to vertical, away from the direction of travel 9003 (FIG. 3D). In an alternative embodiment as shown in FIG. 3E, the adjacent trench delivery system 9000A is shown with the downwardly extending element 9004 having a dogleg or bend forming an upper portion 9020 and a lower portion 9022 with the lower portion 9022 positioned rearward to the direction of travel 9003.

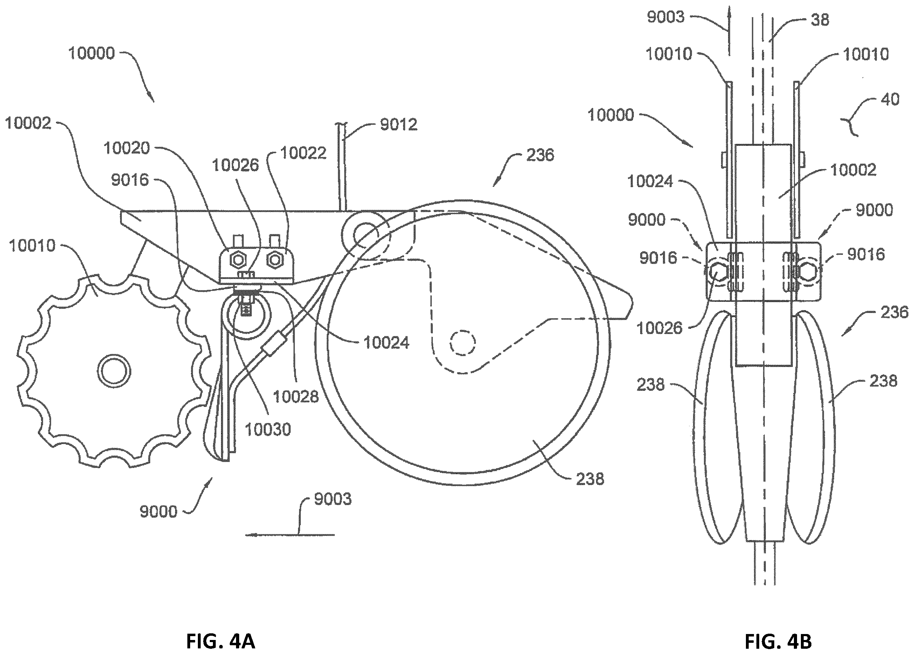

Any of the foregoing embodiments of the adj acent trench liquid delivery system 9000 may be mounted to any component or structure of the planter row unit 200 rearward of the opening system 234 such that liquid is delivered adjacent to the seed trench. As shown in FIGs. 4A-4B, the adjacent trench liquid delivery system 9000 is mounted on assembly frame 10000 forward of the closing wheels 238 of the closing system 236. As shown in FIGs 5A-5B, the adjacent trench liquid delivery system 9000 is mounted on an assembly frame 11000 rearward of the closing wheels 238 of the closing system 236.

Referring to FIGs. 4A-4B, the assembly 10000 is adapted to mount to the planter row unit 200 rearward of the opening assembly 234. The assembly 10000 comprises a frame member 10002 which supports a pair of disc/coulters 10010 which precut a slot in the soil into which the rearwardly aligned adjacent trench liquid delivery system 9000 follows. The disc/coulters 10010 may have a serrated edge or a smooth edge. A mounting bracket 10020 having a vertical flange 10022 and a horizontal flange 10024 is bolted or otherwise rigidly secured to each side of the frame member 10002. The horizontal flange 10024 serves as a horizontal mounting surface for attaching the adjacent trench liquid delivery system 9000 with a bolted connection. As shown, a bolt 10026 extends through an aperture (not shown) in the horizontal flange 10024 and is received through the horizontal loop 9016 of the relief element 9002 on the underside of the horizontal flange. Washers 10028 and a nut 10030 secure the horizontal loop 9016 of the adjacent trench liquid delivery system 9000 to the bolt 10026 and to the mounting bracket 10020. It should be appreciated that the width of mounting bracket 10020 is such that adjacent trench liquid delivery system 9000 will be outside of the trench 38 as best illustrated in the top plan view of FIG. 4B. It should be appreciated that although FIG. 4B shows two adjacent trench liquid delivery systems 9000 mounted to the assembly for depositing liquid on both sides of the seed trench 38, only one adjacent trench deliver system 9000 may be utilized for depositing liquid on either side of the seed trench 38. Additionally, although the frame member 10002 is shown as supporting disc/coulters 10010 and closing wheels 238 of the closing system 236, it should be appreciated that other or additional planter components desired to be mounted rearward of the gauge wheels 248 of the opening assembly 234 may be secured to the frame member 10002 on which the adjacent trench deliver system 9000 is mounted.

In an alternative embodiment shown in FIG. 5A-5B, the adjacent trench delivery system 9000 is shown mounted to another assembly 11000. In this embodiment, the assembly comprises a frame member 11002 to which the adjacent trench delivery system 9000 is mounted after or rearward of the closing wheels 238 of the closing system 236 by a mounting arm 11010 secured thereto. As with the previously described embodiment, two adj acent trench liquid delivery systems 9000 may be supported from the frame member 11002 to deliver liquid to both sides of the seed trench 38, or only one adjacent trench liquid delivery system 9000 may be attached to the frame member 11002 to deliver liquid on either side of the seed trench 38.

In one embodiment, the mounting arm 11010 is U-shaped with a forward end 11012 that bolts to each side of the frame member 11002 and an L-shaped rearward end 11014 forming a horizontal surface 11016. The adjacent trench delivery systems 9000 are attached to the horizontal surface 11016 by bolts 11026 extending through apertures (not shown) in the horizontal surface 10016. The end of the bolt 11026 projecting through the aperture is received through the horizontal loop 9016 of the relief element 9002 on the underside of the horizontal surface 11016. Washers and a nut secure the horizontal loop 9016 of the adjacent trench liquid delivery system 9000 to the bolt 11026 and to the mounting arm 11010.

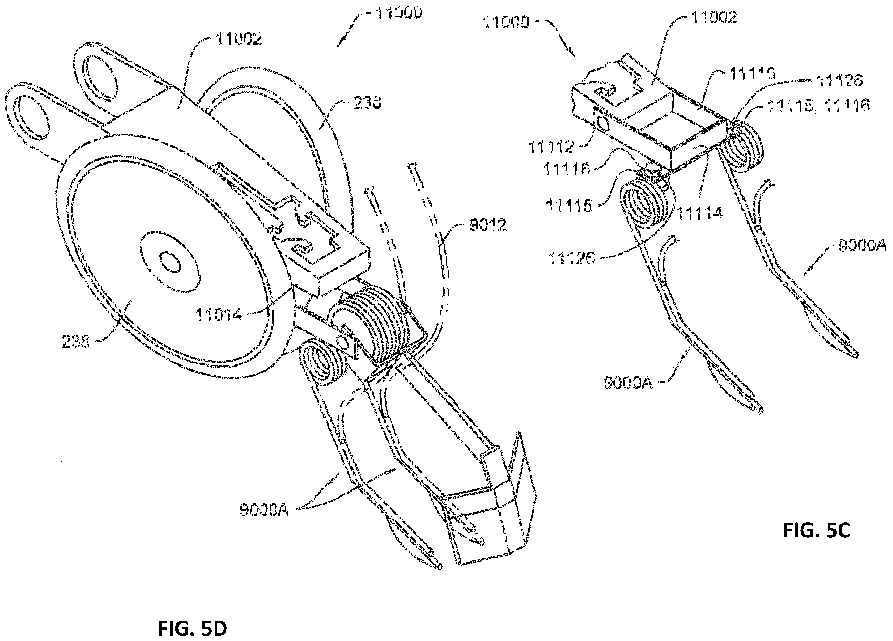

In an alternative embodiment shown in FIG. 5C, mounting arm 11110 includes a U-shaped forward end 11112 that bolts to each side of the assembly frame 11000. The rearward end 11114 of the mounting arm 11110 has outwardly projecting mounting ears 11115 which form the horizontal mounting surface 11116 to which the adjacent trench liquid delivery system 9000 is attached by a bolt 11126 extending through an aperture (not shown) in the horizontal surface 11116. The end of the bolt 11126 projecting through the aperture is received through the horizontal loop 9016 of the relief element 9002 on the underside of the horizontal surface 11116. Washers and a nut secure the horizontal loop 9016 of the adjacent trench liquid delivery system 9000 to the bolt 11126 and to the mounting arm 11110.

It should be appreciated that with either embodiment of the mounting arm 11010 or 11110, the width of the mounting arm is such that adjacent trench liquid delivery system 9000 will be outside of the trench 38 as best illustrated in the top plan view of FIG. 5B.

As shown in FIG. 5D, the mounting arm 11010 or 11110 may be part of a leveling system secured to the frame assembly 11002.

As previously mentioned, any of the various embodiments of the adjacent trench liquid delivery system 9000 shown in FIGs. 3C-3E may be mounted to the assemblies 10000, 11000. It should also be appreciated that the liquid delivered by the adjacent trench liquid delivery system 9000 can be configured to have different liquids delivered to either side of the seed trench 38. The adjacent trench liquid delivery system 9000 may be used alone or in combination with the liquid delivered through any of the firmers described above. An advantage of using them together is that a pass through the field can be eliminated, which reduces soil compaction and damage to growing plants. Also, the liquid can be the same as or different from liquid delivered through any of the firmers described above, thereby allowing for tailoring of liquids (such as fertilizers) that meet the needs of plants at different stages of growth.

Various modifications to the embodiments and the general principles and features of the system and methods described herein and falling within the scope of the invention as defined in the appended claims will be readily apparent to those of skill in the art. Thus, the foregoing description is not to be limited to the embodiments of the apparatus, systems and methods described herein and illustrated in the drawing figures, but is to be accorded the widest scope consistent with the appended claims.

## Claims

1. A liquid application apparatus for applying liquid to soil adjacent to a trench (38) having a first adjacent side and a second adjacent side during a planting operation, comprising:
a planter row unit (200) having an opening system (234) for opening a planting trench (38) in the soil; and
at least one adjacent trench liquid delivery system (9000) mounted to said planter row unit (200) and disposed to inject liquid into soil adjacent to said planting trench (38);
said at least one adjacent trench liquid delivery system (9000) comprising:
a coil spring (9002);
a downwardly extending element (9004), wherein said coil spring (9002) permits said downwardly extending element (9004) to move in a direction along the direction of travel of said planter row unit (200); and
a liquid delivery tube (9010);
**characterized in that**,
said at least one adjacent trench liquid delivery system (9000) is mounted to said planter row unit (200) rearward of said opening system (234), and **in that** the downwardly extending element (9004) comprises a rigid leg (9006) having a knife (9008) disposed on said rigid leg (9006) toward a direction of travel of said planter row unit (200), the liquid delivery tube (9010) being disposed on said rigid leg (9006) rearward of a direction of travel of said planter row unit (200), and wherein the rigid leg is a leg of the coil spring.

2. The liquid application apparatus of claim 1, wherein said coil spring (9002) permits said downwardly extending element (9004) to move in a direction transversely with respect to the direction of travel of said planter row unit.

3. The liquid application apparatus of claim 1, wherein said downwardly extending element (9004) is disposed at an angle from vertical and away from the direction of travel of said planter row unit.

4. The liquid application apparatus of claim 1, wherein said downwardly extending element (9004) includes an upper portion (9020) and a lower portion (9022) extending at an angle from said upper portion (9020) away from the direction of travel of said planter row unit.

5. The liquid application apparatus of claim 1, wherein said knife (9008) has a convex shape toward the direction of travel of said planter row unit.

6. The liquid application apparatus of claim 1, wherein said at least one adjacent trench liquid delivery system (9000) is mounted on an assembly frame (10000;11000) attached to said planter row unit (200) rearward of said opening system (234).

7. The liquid application apparatus of claim 6, wherein said assembly frame (10000;11000) is attached directly behind said opening system (234).

8. The liquid application apparatus of claim 6, wherein said assembly frame (10000) supports at least one disc (10010) in substantial alignment with said at least one adjacent trench liquid delivery system (9000), said at least one disc (10010) disposed to precut the soil ahead of said at least one adjacent trench liquid delivery system (9000).

9. The liquid application apparatus of claim 1, wherein said at least one adjacent trench liquid delivery system (9000) is disposed on said planter row unit (200) rearward of a closing system (236) of said planter row unit.

10. The liquid application apparatus of claim 9, wherein said at least one adjacent trench liquid delivery system (9000) is disposed on a leveling system of said planter row unit.

## Patentansprüche

1. Flüssigkeitsaufbringungsvorrichtung zum Aufbringen von Flüssigkeit auf Boden benachbart einer Furche (38) mit einer ersten benachbarten Seite und einer zweiten benachbarten Seite während eines Pflanz- oder Sävorgangs, mit
einer Pflanz- oder Sämaschinen-Reiheneinheit (200) mit einem Öffnungssystem (234) zum Öffnen einer Pflanz- oder Säfurche (38) in dem Boden; und
mindestens einem benachbarten Furchenflüssigkeitszufuhrsystem (9000), das an der Pflanz- oder Sämaschinen-Reiheneinheit (200) montiert und zum Injizieren von Flüssigkeit in Boden, der der Pflanz- oder Säfurche (38) benachbart ist, angeordnet ist;
wobei das mindestens eine benachbarte Furchenflüssigkeitszufuhrsystem (9000)
eine Schraubenfeder (9002);
ein sich nach unten erstreckendes Element (9004), wobei die Schraubenfeder (9002) es dem sich nach unten erstreckenden Element (9004) erlaubt, sich in einer Richtung längs der Bewegungsrichtung der Pflanz- oder Sämaschinen-Reiheneinheit (200) zu bewegen; und
ein Flüssigkeitszufuhrrohr (9010)
aufweist,
**dadurch gekennzeichnet,**
**dass** das mindestens eine Furchenflüssigkeitszufuhrsystem (9000) hinter dem Öffnungssystem (234) an der Pflanz- oder Sämaschinen-Reiheneinheit (200) montiert ist und dass das sich nach unten erstreckende Element (9004) einen starren Schenkel (9006) mit einem Messer (9008) aufweist, das an dem starren Schenkel (9006) in einer Bewegungsrichtung der Pflanz- oder Sämaschinen-Reiheneinheit (200) angeordnet ist, wobei das Flüssigkeitszufuhrrohr (9010) in einer Bewegungsrichtung der Pflanz- oder Sämaschinen-Reiheneinheit (200) hinten an dem starren Schenkel (9006) angeordnet ist, und wobei der starre Schenkel ein Schenkel der Schraubenfeder ist.

2. Flüssigkeitsaufbringungsvorrichtung nach Anspruch 1, wobei die Schraubenfeder (9002) es dem sich nach unten erstreckenden Element (9004) erlaubt, sich in einer Richtung quer zu der Bewegungsrichtung der Pflanz- oder Sämaschinen-Reiheneinheit zu bewegen.

3. Flüssigkeitsaufbringungsvorrichtung nach Anspruch 1, wobei das sich nach unten erstreckende Element (9004) unter einem Winkel zur Vertikalen und weg von der Bewegungsrichtung der Pflanz- oder Sämaschinen-Reiheneinheit angeordnet ist.

4. Flüssigkeitsaufbringungsvorrichtung nach Anspruch 1, wobei das sich nach unten erstreckende Element (9004) einen oberen Bereich (9020) und einen unteren Bereich (9022), der sich unter einem Winkel von dem oberen Bereich (9020) weg von der Bewegungsrichtung der Pflanz- oder Sämaschinen-Reiheneinheit erstreckt, umfasst.

5. Flüssigkeitsaufbringungsvorrichtung nach Anspruch 1, wobei das Messer (9008) zu der Bewegungsrichtung der Pflanz- oder Sämaschinen-Reiheneinheit hin eine konvexe Form aufweist.

6. Flüssigkeitsaufbringungsvorrichtung nach Anspruch 1, wobei das mindestens eine benachbarte Furchenflüssigkeitszufuhrsystem (9000) an einem Montagerahmen (10000; 11000) montiert ist, der hinter dem Öffnungssystem (234) an der Pflanz- oder Sämaschinen-Reiheneinheit (200) befestigt ist.

7. Flüssigkeitsaufbringungsvorrichtung nach Anspruch 6, wobei der Montagerahmen (10000; 11000) unmittelbar hinter dem Öffnungssystem (234) befestigt ist.

8. Flüssigkeitsaufbringungsvorrichtung nach Anspruch 6, wobei der Montagerahmen (10000) mindestens eine Scheibe (10010) im Wesentlichen Flucht mit dem mindestens einen benachbarten Furchenflüssigkeitszufuhrsystem (9000) abstützt, wobei die mindestens eine Scheibe (10010) angeordnet ist, um den Boden vor dem mindestens einen benachbarten Furchenflüssigkeitszufuhrsystem (9000) vorzuschneiden.

9. Flüssigkeitsaufbringungsvorrichtung nach Anspruch 1, wobei das mindestens eine benachbarte Furchenflüssigkeitszufuhrsystem (9000) an der Pflanz- oder Sämaschinen-Reiheneinheit (200) hinter einem Schließsystem (236) der Pflanz- oder Sämaschinen-Reiheneinheit angeordnet ist.

10. Flüssigkeitsaufbringungsvorrichtung nach Anspruch 9, wobei das mindestens eine benachbarte Furchenflüssigkeitszufuhrsystem (9000) an einem Niveauregulierungssystem der Pflanz- oder Sämaschinen-Reiheneinheit angeordnet ist.

## Revendications

1. Dispositif d'application de liquide destiné à appliquer un liquide sur le sol de manière adjacente à une tranchée (38) présentant un premier côté adjacent et un deuxième côté adjacent au cours d'une opération de plantation, comprenant :
une unité de formation de rangée de plantation (200) comportant un dispositif d'ouverture (234) destiné à ouvrir une tranchée de plantation (38) dans le sol ; et
au moins un dispositif d'alimentation de liquide de tranchée adjacent (9000) monté sur ladite unité de formation de rangée de plantation (200) et disposé afin d'injecter un liquide dans le sol de manière adjacente à ladite tranchée de plantation (38) ;
ledit au moins un dispositif d'alimentation de liquide de tranchée adjacent (9000) comprenant:
un ressort hélicoïdal (9002) ;
un élément s'étendant vers le bas (9004), dans lequel ledit ressort hélicoïdal (9002) permet le déplacement dudit élément s'étendant vers le bas (9004) dans une direction suivant la direction de déplacement de ladite unité de formation de rangée de plantation (200) ; et
un tube d'alimentation de liquide (9010) ;
**caractérisé en ce que**,
ledit au moins un dispositif d'alimentation de liquide de tranchée adjacent (9000) est monté sur ladite unité de formation de rangée de plantation (200) vers l'arrière dudit dispositif d'ouverture (234), et **en ce que** l'élément s'étendant vers le bas (9004) comprend une patte rigide (9006) comportant un couteau (9008) disposé sur ladite patte rigide (9006) suivant une direction de déplacement de ladite unité de formation de rangée de plantation (200), le tube d'alimentation de liquide (9010) étant disposé sur ladite patte rigide (9006) vers l'arrière suivant une direction de déplacement de ladite unité de formation de rangée de plantation (200), et dans lequel la patte rigide est une patte du ressort hélicoïdal.

2. Dispositif d'application de liquide selon la revendication 1, dans lequel ledit ressort hélicoïdal (9002) permet le déplacement dudit élément s'étendant vers le bas (9004) dans une direction transversale par rapport à la direction de déplacement de ladite unité de formation de rangée de plantation.

3. Dispositif d'application de liquide selon la revendication 1, dans lequel l'élément s'étendant vers le bas (9004) est disposé suivant un certain angle par rapport à la verticale et à l'opposé de la direction de déplacement de ladite unité de formation de rangée de plantation.

4. Dispositif d'application de liquide selon la revendication 1, dans lequel l'élément s'étendant vers le bas (9004) comporte une partie supérieure (9020) et une partie inférieure (9022) s'étendant sous un certain angle par rapport à ladite partie supérieure (9020) à l'opposé de la direction de déplacement de ladite unité de formation de rangée de plantation.

5. Dispositif d'application de liquide selon la revendication 1, dans lequel ledit couteau (9008) présente une forme convexe dans la direction de déplacement de ladite unité de formation de rangée de plantation.

6. Dispositif d'application de liquide selon la revendication 1, dans lequel ledit au moins un dispositif d'alimentation de liquide de tranchée adjacent (9000) est monté sur un châssis d'ensemble (10000 ; 11000) fixé sur ladite unité de formation de rangée de plantation (200) vers l'arrière dudit dispositif d'ouverture (234).

7. Dispositif d'application de liquide selon la revendication 6, dans lequel ledit châssis d'ensemble (10000;11000) est fixé directement derrière ledit dispositif d'ouverture (234).

8. Dispositif d'application de liquide selon la revendication 6, dans lequel ledit châssis d'ensemble (10000) supporte au moins un disque (10010) sensiblement dans l'alignement avec ledit au moins un dispositif d'alimentation de liquide de tranchée adjacent (9000), ledit au moins un disque (10010) étant disposé de manière à pré-découper le sol à l'avant dudit au moins un dispositif d'alimentation de liquide de tranchée adjacent (9000).

9. Dispositif d'application de liquide selon la revendication 1, dans lequel ledit au moins un dispositif d'alimentation de liquide de tranchée adjacent (9000) est disposé sur ladite unité de formation de rangée de plantation (200) vers l'arrière d'un dispositif de fermeture (236) de ladite unité de formation de rangée de plantation.

10. Dispositif d'application de liquide selon la revendication 9, dans lequel ledit au moins un dispositif d'alimentation de liquide de tranchée adjacent (9000) est disposé sur un dispositif de nivelage de ladite unité de formation de rangée de plantation.
